(19) Europäisches Patentamt / European Patent Office / Office européen des brevets

(11) **EP 3 417 778 A1**

(12) **EUROPÄISCHE PATENTANMELDUNG**

(43) Veröffentlichungstag:
**26.12.2018  Patentblatt 2018/52**

(51) Int Cl.:
**A61B 5/12** (2006.01)    **A61B 5/0484** (2006.01)
**A61B 5/00** (2006.01)    *A61B 5/16* (2006.01)

(21) Anmeldenummer: **18000549.8**

(22) Anmeldetag: **21.06.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **22.06.2017   DE 102017005867**

(71) Anmelder:
• **Universität des Saarlandes**
  **66123 Saarbrücken (DE)**
• **Hochschule RheinMain**
  **65197 Wiesbaden (DE)**

(72) Erfinder:
• **Hecker, Dietmar**
  **66501 Kleinbundenbach (DE)**
• **Metzler, Patrick**
  **66606 St. Wendel (DE)**
• **Schick, Bernhard**
  **36145 Hofbieber (DE)**
• **Schorn, Bianca**
  **48147 Münster (DE)**

(74) Vertreter: **Nobis, Wolfgang**
  **Fritz-Häuser-Strasse 29**
  **71522 Backnang (DE)**

(54) **AUTOMATISIERTES VERFAHREN ZUR VERRINGERUNG DES STÖRSIGNALEINFLUSSES BEI DER ANALYSE VON EVOZIERTEN POTENZIAL-SIGNALEN ODER EREIGNISKORRELIERTEN POTENZIAL-SIGNALEN EINER NEURONALEN AKTIVITÄT**

(57)    Automatisiertes Verfahren zur Verringerung des Störsignaleinflusses bei der Analyse von evozierten Potenzial-Signalen oder ereigniskorrelierten Potenzial-Signalen einer neuronalen Aktivität

Die Erfindung gibt ein automatisiertes Verfahren zur Verringerung eines Störeinflusses bei der Analyse von evozierten Potenzial-Signalen oder ereigniskorrelierten Potenzial-Signalen einer neuronalen Aktivität an. Es weist auf:
- Darbieten von physiologischen Reizen einem Testsubjekt (2), wobei der physiologische Reiz durch mindestens einen Parameter (RP) beschreibbar ist,
- Ermitteln von abgeleiteten zeitabhängigen Potenzial-Signalen,
- Definieren einer ersten und/oder zweiten Klasse,
- Zuordnen der Potenzial-Signale oder Teilabschnitten der Potenzial-Signale zu der ersten Klasse oder der zweiten Klasse durch eine Klassierungseinheit,
- Ermitteln von Häufigkeiten der der ersten Klasse zugeordneten und/oder der zweiten Klasse zugeordneten Potenzial-Signale oder zugehörigen

Eine zugehörige Anordnung und ein zugehöriges computerlesbares Speichermedium werden ebenfalls angegeben.

FIG 3

EP 3 417 778 A1

**Beschreibung**

Automatisiertes Verfahren zur Verringerung des Störsignaleinflusses bei der Analyse von evozierten Potenzial-Signalen oder ereigniskorrelierten Potenzial-Signalen einer neuronalen Aktivität

Gebiet der Erfindung

Die Erfindung betrifft ein automatisiertes Verfahren zur Verringerung des Störsignaleinflusses bei der Analyse von evozierten Potenzial-Signalen oder ereigniskorrelierten Potenzial-Signalen einer neuronalen Aktivität, eine Anordnung zur Verringerung des Störsignaleinflusses bei der Analyse von evozierten Potenzial-Signalen oder ereigniskorrelierten Potenzial-Signalen einer neuronalen Aktivität sowie ein computerlesbares Medium zur Durchführung des automatisierten Verfahrens. Die Erfindung kann für nicht-diagnostische und diagnostische Anwendungen eingesetzt werden.

Hintergrund der Erfindung

Evozierte und ereigniskorrelierte Potenziale sind ein etabliertes Werkzeug der objektiven neurologischen Diagnostik. Weltweit werden in Kliniken verschiedenster Fachrichtungen täglich viele medizinische Entscheidungen aufgrund von evozierten und ereigniskorrelierten Potenzialen gefällt. Evozierte und ereigniskorrelierte Potenziale (EP und EKP) bezeichnen Potenzialunterschiede bzw. Wellenformen beispielsweise im Elektroenzephalogramm (EEG), die entweder durch Reizung eines Sinnesorgans bzw. eines peripheren Nervs ausgelöst werden oder mit kognitiven Prozessen, wie beispielsweise Aufmerksamkeit oder Sprachverarbeitung, korreliert sind. Mit dem Begriff EKP werden im Folgenden evozierte, ereigniskorrelierte und induzierte Potenziale zusammengefasst.

EKPs haben im EEG eine sehr kleine Amplitude von einigen nV bis wenigen $\mu$V. Um daher die EKPs im ereignisunabhängigen Spontan-EEG mit einer Amplitude von etwa 50 bis 100 $\mu$V sichtbar machen zu können, müssen die gemessenen EEG-Signale mit Hilfe von Methoden der Signalanalyse ausgewertet werden. Dazu werden im einfachsten Fall mehrere Realisierungen eines EKPs gemittelt. Das Spontan-EEG wird dabei als stochastisches Störsignal betrachtet, das von dem Reiz oder dem kognitiven Prozess unabhängig und dessen Mittelwert Null ist und begrenztem Energieinhalt. Hingegen sind die interessierenden EKPs zeitlich an den Reiz bzw. dem kognitiven Prozess gekoppelt.

Das EKP zeigt nach jedem Reiz bzw. kognitiven Prozess nahezu den gleichen Verlauf. Durch die wiederholte Darbietung eines Reizes bzw. die Wiederholung eines kognitiven Prozesses und die Mittelung des nachfolgenden EEG-Segments strebt die unabhängige Aktivität gegen Null, während das EKP aufsummiert wird. Die Anzahl der in der Praxis notwendigen Realisierungen hängt von dem Signal-Rausch-Verhältnis ab und ist je nach Sinnesmodalität und physikalischen Charakteristika verschieden. Bei beispielsweise einem durch Lichtblitze ausgelösten Potenzial genügen etwa 50 Reize, während zur Messung der frühen akustischen Hirnstammpotenziale deutlich mehr Reize dargeboten werden müssen. Die Auswertung der EKPs berücksichtigt die Form der Welle, die Wellenhöhe (Amplitude) und die Laufzeit (Latenz).

Je nachdem, wie lange nach einem Ereignis eine Komponente im EEG auftritt, kann man diese verschiedenen Gehirnregionen zuordnen. Grob gilt folgende Einteilung: frühe Komponenten (0 bis 10 ms), mittlere Komponenten (10 bis 50 ms) und späte oder langsame Komponenten (50 bis 1000 ms).

Um die Signalauswertung der EKPs zu verbessern, werden seit geraumer Zeit Methoden der Signalanalyse verwendet. Mit ihnen lassen sich beispielweise Veränderungen in den Schwingungsstärken (Zeit-Frequenz-Analyse, Wavelet-Analyse), in der Synchronisation oder in der Kohärenz über die Einzelmessungen nachweisen. In der Offenlegungsschrift DE 10 2014 007 647 A1 wird beispielsweise ein relativ komplexes Verfahren der Signalanalyse mit Hilfe der Momentanphasen von Einzelsweep-Signalen beschrieben.

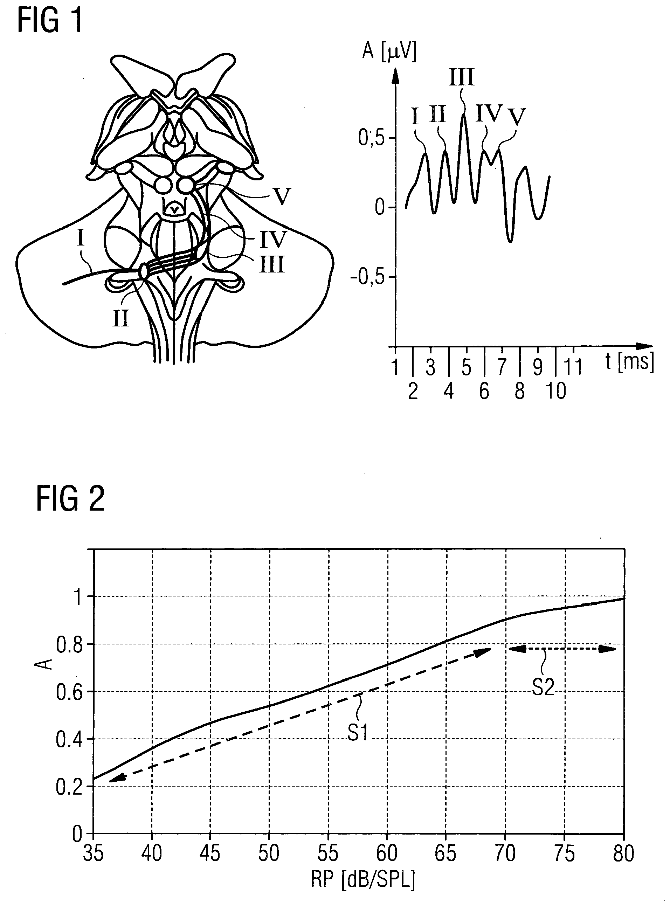

Frühe Komponenten von EKPs spielen bei der Frage nach dem objektiven Hörvermögen eine große Rolle. Eine entsprechende Diagnostik stützt sich dabei auf die Messung der frühen akustisch evozierten Hirnstamm - Potenziale (brainstem evoked response audiometry = BERA). Während mit otoakustischen Emissionen die Funktionsfähigkeit der äußeren Haarzellen (OHC) überprüft werden kann, sind es bei einer BERA-Messung die inneren Haarzellen (IHC) und die Anteile der zentralem Hörbahn (= Hirnstamm). Der Hirnstamm **(Fig. 1)** befindet sich anatomisch zwischen dem Rückenmark und dem Großhirn. Er kontrolliert dabei Blutdruck und Herzfrequenz, steuert Atmung bzw. Schwitzen und reguliert Wachsein und Schlafen bis ins Detail.

Als entscheidende Schaltzentrale werden auch lebenswichtige Reflexe (vestibulookulärer Reflex, Kornealreflex, Pupillenreflex, Trachealreflex, Pharyngealreflex) über ihn geführt. Durch diese Zuordnung von vitalen bzw. basalen Funktionen, sind Potenziale, die vom Hirnstamm ausgelöst werden, relativ narkoseunabhängig. Entsprechend kann eine BERA-Messung unter Narkose durchgeführt werden. Dies wird oftmals bei Kleinkindern bzw. Kindern genutzt, um objektive Resultate über ihr Hörvermögen, z.B. zur Einstellung von Hörhilfen, zu erhalten. Bei der akustischen Reizung des Ohres (meist über Kopfhörer) werden verschiedene Kerngebiete im Hirnstamm angeregt und deren Reaktion kann als Potenzial-Signal (= Potenzialwelle) in einer BERA-Messung registriert werden.

Die einzelnen Stationen werden mit den römischen Zahlen I bis VI benannt. Allerdings geht man davon aus, dass diese Wellen nicht starr einer Station zugeschrieben werden können, sondern vielmehr als komplexe Überlagerung verschiedener Generatoraktivitäten verstanden werden. Wie in der gemittelten Kurve in **Fig. 1** (dargestellt ist die Signalstärke A in µV in Abhängigkeit der Zeit t in ms) zu sehen ist, stehen die Wellenkomplexe I bis V in einem festen Zeitfenster von 0 bis 10 ms. Dabei bezeichnet

I: den peripheren Anteil des Nervus chochleans,
II: den zentralen Anteil des Nervus chochleans,
III: den Anteil des Nucleus chochleans,
IV: den Anteil des Nucleus olivaris sauperior und
V: den Anteil des Colliculi inferiores.

Zur Registrierung dieser Wellenverläufe werden dem Patienten drei oder vier Elektroden am Kopf platziert mit denen ein EEG aufgezeichnet wird. Parallel zur Aufzeichnung werden zur Stimulation des Gehörs zeitgleich über einen Kopfhörer definierte akustische Stimuli (= akustischer Reiz) appliziert. Dies sind oftmals Click-Reize oder Töne definierter Frequenz (tone-bursts) im Bereich von 500 Hz bis 4.000 Hz. Da hierbei extrem niedrige EEG Potenziale (siehe oben) gemessen werden, wird das Potenzial-Signal entsprechend oft gemittelt, um einen ausreichenden Signal-Rauschabstand (SNR) zu erhalten. Bei einer solchen Mittelung (Averaging) der Potenzial-Signale werden standardmäßig 2.000 gleichförmige Reize wiederholt dargeboten und die zugehörigen Potenzial-Signale, auch Single-Sweeps genannt, gemessen und kontinuierlich gemittelt.

Durch ein derartiges Verfahren werden morphologische Signale verstärkt und zufälliges Rauschen verringert. Aus BERA - Messungen des klinischen Alltags ist bekannt, dass meist nur die Wellen I, III und V dominant und sichtbar sind. Des Weiteren zeigen BERA-Messungen, dass laute Stimuli hohe und leise Stimuli kleine Potenziale reproduzieren. Aus diesen Parametern kann eine Laustärken - Wachstumsfunktion ermittelt werden. Weiterhin ist bekannt, dass sich die Latenzen der Wellen auch in Abhängigkeit des Schalldruckpegels verändern: laute Reize werden früher, leise Reize später abgebildet.

Auswertungen von BERA-Messungen erfolgen auf visuelle Art durch einen Facharzt bzw. Audiologen. Merkmale dieser Auswertung sind Latenz und Hörschwelle, d.h. bis zu welchem Reizpegel lässt sich ein Wellenkomplex noch erkennen. Prinzipiell lässt sich mit einer BERA-Messung eine Abschätzung der Hörschwelle durchführen. Dies ist jedoch oftmals nicht einfach. In einigen Fällen (beispielsweise Tinnitus-Patient) ist ein Untersucher geneigt, Wellen zu detektieren, deren Ursprung nicht auf der physiologischen Reizantwort liegt, sondern auf Wellenformationen beruhen, deren kortikale Ausprägungen BERAwellenförmig verhalten.

Im klinischen Alltag der Anmelderinnen sind bis heute nur Geräte bekannt, mit deren Hilfe die Hörschwelle visuell abgeschätzt werden muss. Auch wird das Messergebnis durch ein Mittelungsverfahren generiert. Dabei können maximal drei Kurven erzeugt werden: einmal mit n Messungen und zweimal mit n/2 Messungen. Eine Singlesweepanalyse ist nicht möglich.

Klinisch angewandte Verfahren nutzen lediglich die Mittelungstechnik bzw. treffen Entscheidungen bei deutlich überschwelligem Reiz beim Hörscreening von Neugeborenen. Messdaten müssen durch einen erfahrenen Facharzt oder Audiologen ausgewertet werden. Derzeit gibt es nur eine schwellen- und latenzbezogene Auswertung. Oftmals falsch ausgewertet BERA-Messungen können schwerwiegende Konsequenzen haben. Auch ist es bis heute nicht möglich, auffällige Morphologien in BERA-Messungen zu klassifizieren.

Bestimmungen der Hörschwelle mittels abgeleiteter Potenzial-Signale werden beispielsweise in den Patentanmeldungen WO 2015/161343 A1 und WO 2008/065239 A1 offenbart.

Zusammenfassung der Erfindung

Es ist Aufgabe der Erfindung, eine Lösung anzugeben, die den Einfluss von insbesondere stochastischen Störsignalen bei der Analyse von evozierten und ereigniskorrelierten Potenzial-Signalen von neuronalen Aktivitäten vermindert.

Gemäß der Erfindung wird die gestellte Aufgabe mit den Verfahren, der Anordnung und dem computerlesbaren Speichermedium der unabhängigen Patentansprüche gelöst.

Erfindungsgemäß werden evozierte Potenzial-Signale oder ereigniskorrelierte Potenzial-Signalen einer neuronalen Aktivität, wie folgt automatisiert ausgewertet:

- einem Testsubjekt (Mensch oder Tier) werden physiologische Reize beispielsweise akustische Schallereignisse) dargeboten, wobei der physiologische Reiz durch mindestens einen Parameter (wie beispielsweise die Lautstärke oder Frequenz) charakterisiert ist,
- abgeleitete zeitabhängigen Potenzial-Signale werden beispielsweise mittels Elektroenzephalografie oder Magnetoenzephalografie ermittelt,

- eine erste und/oder eine zweite Klasse werden definiert,
- die Potenzial-Signale oder auch nur Teilabschnitte der Potenzial-Signale werden einer ersten Klasse oder einer zweiten Klasse zugeordnet (= Klassieren) und
- die Anzahl der der ersten Klasse richtig zugeordneten und/oder der zweiten Klasse richtig zugeordneten Potenzial-Signale oder zugehörigen Teilabschnitten der Potenzial-Signale wird bestimmt..

Die Erfindung beansprucht ein automatisiertes (computerimplementiertes) Verfahren zur Verringerung eines Störsignaleinflusses bei der Analyse von evozierten Potenzial-Signalen oder ereigniskorrelierten Potenzial-Signalen einer neuronalen Aktivität. Es weist folgende Schritte auf:

- Darbieten von physiologischen Reizen einem Testsubjekt, wobei der physiologische Reiz durch mindestens einen Parameter beschreibbar ist,
- Ermitteln von abgeleiteten zeitabhängigen Potenzial-Signalen,
- Definieren einer ersten und/oder zweiten Klasse,
- Zuordnen (Klassieren) der Potenzial-Signale oder Teilabschnitten der Potenzial-Signale zu der ersten Klasse oder der zweiten Klasse durch eine Klassierungseinheit,
- Ermitteln von Häufigkeiten der der ersten Klasse richtig zugeordneten und/oder der zweiten Klasse richtig zugeordneten Potenzial-Signale oder zugehörigen Teilabschnitten der Potenzial-Signale und/oder
- Ermitteln von Häufigkeiten der der ersten Klasse falsch zugeordneten und/oder der zweiten Klasse falsch zugeordneten Potenzial-Signale oder zugehörigen Teilabschnitten der Potenzial-Signale.

Die Erfindung beansprucht auch ein automatisiertes Verfahren zur Verringerung eines Störsignaleinflusses bei der Analyse von evozierten Potenzial-Signalen oder ereigniskorrelierten Potenzial-Signalen einer neuronalen Aktivität. Es weist folgende Schritte auf:

- Darbieten von physiologischen Reizen einem Testsubjekt, wobei der physiologische Reiz durch mindestens einen Parameter beschreibbar ist,
- Ermitteln von abgeleiteten zeitabhängigen Potenzial-Signalen,
- Definieren einer ersten und/oder zweiten Klasse,
- Zuordnen der Potenzial-Signale oder Teilabschnitten der Potenzial-Signale zu der ersten Klasse oder der zweiten Klasse durch eine Klassierungseinheit und
- Ermitteln von Häufigkeiten der der ersten Klasse zugeordneten und/oder der zweiten Klasse zugeordneten Potenzial-Signale oder zugehörigen Teilabschnitten der Potenzial-Signale.

In einer Weiterbildung des Verfahrens wird eine erste Vergleichsfunktion bereitgestellt, wobei die erste Klasse als zur Klasse der ersten Vergleichsfunktion gehörig und die zweite Klasse als zur Klasse einer zweiten Vergleichsfunktion gehörig oder nicht zur Klasse der ersten Vergleichsfunktion gehörig definiert ist. Die zweitet Vergleichsfunkton kann eine stochastischen Zufallsverteilungsfunktion oder einer konstanten Funktion gleichen Mittelwerts sein.

In einer weiteren Ausgestaltung, wird obiges Verfahren wiederholt durchgeführt, wobei der Wert des Parameters vor jedem wiederholten Durchführen verändert wird.

In einer weiteren Ausgestaltung werden die ermittelten Häufigkeiten in Abhängigkeit des Wertes des Parameters beispielsweise auf einem Bildschirm dargestellt.

In einer weiteren Ausführung wird ein Schwellwert des Parameters ermittelt, ab dem die Häufigkeiten von einem vorgebbaren Häufigkeitsschwellwert, vorzugsweise 50 %, oder um eine vorgebbare Toleranz um den Häufigkeitsschwellwert abweichen.

In einer weiteren Ausgestaltung kann die erste Vergleichsfunktion aus mindestens einem Potenzial-Signal des Testsubjekts ermittelt werden.

In einer weiteren Ausgestaltung kann die erste und/oder zweite Vergleichsfunktion aus mindestens einem Potenzial-Signal des Testsubjekts ohne Darbietung eines physiologischen Reizes ermittelt werden.

In einer weiteren Ausgestaltung kann die erste und/oder zweite Vergleichsfunktion aus Potenzial-Signalen mindestens eines weiteren Testsubjekts ermittelt werden.

In einer weiteren Ausgestaltung kann die erste und/oder zweite Vergleichsfunktion durch Mittelwertbildung von Potenzial-Signalen oder durch Verfahren der künstlichen Intelligenz ermittelt werden.

In einer Weiterbildung kann das Klassieren entsprechend der kleinsten Summe der Fehlerquadrate (unter Benutzung der euklidischen Distanz) bezogen auf die erste Vergleichsfunktion und die zweite Vergleichsfunktion erfolgen.

In einer weiteren Ausprägung können der physiologische Reiz ein akustischer Reiz, der Parameter ein Schalldruckpegel und der Schwellwert eine Hörschwelle sein.

Die Erfindung beansprucht auch eine Anordnung zur Verringerung eines Störsignaleinflusses bei der Analyse von evozierten Potenzial-Signalen oder ereigniskorrelierten Potenzial-Signalen einer neuronalen Aktivität. Die Anordnung weist auf:

- eine Stimuli-Generatoreinheit, die ausgebildet ist, mindestens einen physiologischen Reiz darzubieten,
- eine Signalerfassungseinheit mit mindestens einem Sensor, die ausgebildet ist, eine neuronale Aktivität aufgrund des physiologischen Reizes zu erfassen, und
- eine eine Klassierungseinheit aufweisende Rechen- und Steuereinheit, die ausgebildet und programmiert ist, das erfindungsgemäße Verfahren auszuführen.

Außerdem beansprucht die Erfindung ein computerlesbares Medium, auf welchem ein Computerprogramm gespeichert ist, wobei das Computerprogramm in eine Speichereinrichtung einer Einrichtung ladbar ist, wobei mit dem Computerprogramm die Schritte eines erfindungsgemäßen Verfahrens ausgeführt werden, wenn das Computerprogramm auf der Einrichtung ausgeführt wird.

Ein Vorteil der Erfindung ist die automatisierte Ermittlung der Hörschwelle, ohne a priori Wissen. Außerdem sind die zu berücksichtigenden Wellenabschnitte der Potenzial-Signale frei einstellbar (komplettes Signal oder nur ein Wellenkomplex). Bei der Analyse können darüber hinaus unterschiedliche Wellenformen unterschiedlichen Krankheiten (z.B. ADHS, AVWS, Autismus, Tinnitus, Neuropathie von Synaptopathie unterscheiden) zugeordnet werden. Auswirkungen verschiedener Gendefekte auf das Hören können fest gestellt werden (novelty detection). Mit der Erfindung ist es erstmals möglich, nicht nur die Hörschwelle zu bestimmen, sondern Auffälligkeiten im kompletten Wellenkomplex darzustellen und zu definieren.

Weitere Besonderheiten und Vorteile der Erfindung werden aus den nachfolgenden Erläuterungen von Ausführungsbeispielen und anhand von schematischen Zeichnungen ersichtlich.

Es zeigen:

Fig. 1: einen Hirnstamm und ein Schaubild eines Potenzial-Signals,

Fig. 2: ein Schaubild einer Amplitudenwachstumsfunktion eines Testsubjekts,

Fig. 3: ein Schaubild der CCR- und MCR-Funktionen des Testsubjekts,

Fig. 4: ein Schaubild der CCR- und MCR-Funktionen eines weiteren Testsubjekts und

Fig. 5: ein Blockschaltbild einer Anordnung zur Durchführung eines Verfahrens zur Analyse akustische evozierter Potenzial-Signale.

Detaillierte Beschreibung von Ausführungsbeispielen

Im Folgenden wird die Erfindung beispielhaft anhand einer Anwendung für akustische Reize und EEG beschrieben.

Fig. 2 zeigt die Anwendung einer Analysesoftware der Anmelderinnen zur visuellen Auswertung der Hörfunktion an einem Tiermodell (= Testsubjekt) mittels einer Amplitudenwachstumsfunktion in einem Schaubild. Dargestellt ist die normierte Amplitude A einer Welle über dem Schalldruckpegel RP in dB/SPL. Zu erkennen ist ein Bereich S1 der mit dem Schalldruckpegel RP anwächst und ein Sättigungsbereich S2. Die aufwendig und subjektiv bestimmbare Amplitudenwachstumsfunktion stellt die Amplitudenhöhen über den Reizschwellen dar. Diese Funktion gibt Auskunft über die neuronale Verarbeitung von empfunden Lautstärken. Neben der Hörschwelle können noch Amplituden und Latenzen der einzelnen Wellen bestimmt werden.

Die verwendete Software wurde von den Anmelderinnen entwickelt. Üblicher Weise werden diese Parameter im klinischen Forschungsbereich per Hand ausgelesen und in eine Excel-Tabelle übertragen. Die von den Anmelderinnen verwendete Software automatisiert dies weitgehend, basiert aber trotzdem auf einer visuellen Auswertung von BERA-Messungen.

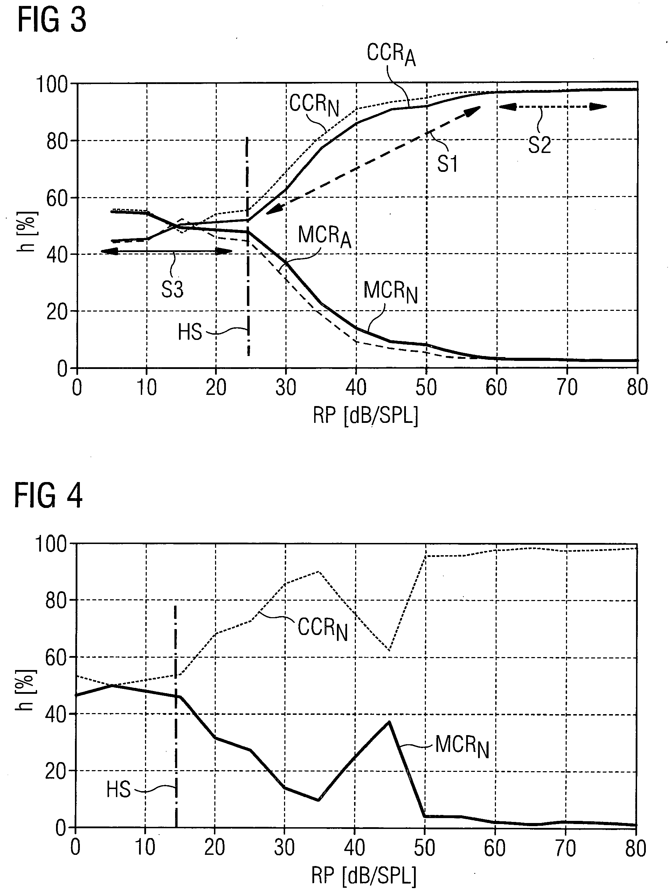

Hier setzt eine erfindungsgemäße Lösung an, indem Hörschwellen HS durch CR-Funktionen CCR und MCR (CCR = Correct Classification Rate; MCR = MisClassification Rate) ermittelt werden, wobei die Amplitudenwachstumsfunktion gemäß Fig. 2 ersetz wird. Die CCR-Funktionen, wie im Schaubild der Fig. 3 dargestellt, werden wie folgt ermittelt.

Die Singlesweeps (= Potenzial-Signale) einer Messung werden einer ersten Klasse oder einer zweiten Klasse zugeordnet (= Klassieren). Die erste Klasse entspricht der Nullhypothese (mit Index N bezeichnet), dass der Singlesweep zu einer physiologischen Reizantwort (= erste Vergleichsfunktion) des Patienten bzw. zu einer Untergruppe einer Normgruppe gehört. Die andere Klasse entspricht der Antithese (mit Index A bezeichnet), dass die Reizantwort dem Antwortverhalten einer stochastischen Größe bzw. einer konstanten Funktion gleichen Mittelwertes (= zweite Vergleichsfunktion) entspricht.

Werden als Referenz eine Konstante, oder die Singlesweeps bei niedriger Reizschwelle gewählt, ersetzen die CCR-Funktionen $CCR_N$ und $CCR_A$ die Wachstumsfunktion. **Fig. 3** zeigt das Schaubild der relativen Häufigkeiten h in % in Abhängigkeit der Schalldruckpegel RP in dB/SPL. Deutlich zu erkennen sind ein ansteigender Bereich S1, ein Sättigungsbereich S2 und ein Bereich S3 unterhalb der Hörschwelle HS.

Entscheidungsregeln basierend auf Trainingsdaten erlauben eine Zuordnung ohne Testdaten, bzw. ohne die wahren Daten zu kennen. Durch Vergleich mit der wahren Zuordnung lässt sich ein Fehlermaß für die regelbasierte Zuordnung finden. Der Verlauf des Zuordnungsfehlers über der Reizschwelle (= Hörschwelle HS) sind die MCR Funktionen $MCR_N$ und $MCR_A$. Der nächstgelegene Wert unterhalb der 50% Fehlergrenze (= Häufigkeitsschwellwert) bestimmt beispielsweise die Hörschwelle HS.

Das Zuordnen (Klassieren) kann am einfachsten entsprechend der kleinsten Summe der Fehlerquadrate bezogen auf die erste Vergleichsfunktion die zweite Vergleichsfunktion erfolgen. Weitere anwendbare Verfahren werden weiter unten beschrieben.

Und **Fig. 4** zeigt ein Schaubild eines weiteren Tiermodells (= Testsubjekt) mit einer CCR-Funktion $CCR_N$ und einer MCR-Funktion $MCR_N$ ähnlich **Fig. 3**. Dargestellt sind wiederum die realtiven Häufigkeiten h in % in Abhängigkeit des Schalldruckpegels RP in dB/SPL. Deutlich zu erkennen ist neben der Hörschwelle HS ein Höreinbruch bei etwa 45 dB/SPL. Dieser wäre so bei den oben beschriebenen bekannten BERA-Auswertungen nicht oder nur schwer erkannt worden.

Referenziert man auf eine Norm- oder Pathogruppe (z.B. Autismus, ADHS, Down Syndrome, Tinnitus, etc.) die neu gewonnenen Merkmale eines unbekannten Patienten, so kann dieser mit der CCR- oder MCR-Funktion eindeutig zugewiesen werden.

Bei einem Vergleich mit einem weiteren Testsubjekt wird wie folgt vorgegangen. Zunächst wird die zwischen einem gesunden Testsubjekt und einem Testsubjekt einer bekannten Erkrankung gefundene Entscheidungsregel benutzt und auf die Singlesweeps des weiteren Testsubjekts angewendet. Die CCR oder MCR-Funktion kann dann nicht unmittelbar angegeben werden, da man nicht weiß, ob das weitere Testsubjekt in Wirklichkeit der ersten Klasse oder der zweiten Klasse angehört.

Man kann aber eine CCR-Funktion unter der Annahme, dass das weitere Testsubjekt zur ersten Klasse gehört, definieren. Unter der Annahme, dass auch die Unterschiede zwischen dem Testsubjekt und dem Testsubjekt mit der bekannten Erkrankung mit der Reizstärke zunehmen, lässt sich die These, dass das weitere Testsubjekt zur ersten Klasse gehört überprüfen, indem man schaut, ob CCR_WennKlasse1 ansteigt.

In einer weiteren erfindungsgemäßen Lösung werden die Häufigkeiten der der ersten Klasse zugeordneten und/oder der zweiten Klasse zugeordneten Potenzial-Signale oder zugehörigen Teilabschnitten der Potenzial-Signale ermittelt. Die Klassierungseinheit 8 wird dabei so trainiert, dass beispielsweise die Zuordnung zur Klasse einer ersten oder zweiten Vergleichsfunktion mit einer Wahrscheinlichkeit von 50 % erfolgt, wenn kein akustischer Reiz oder ein unterschwelliger akustischer Reiz dargeboten wird.

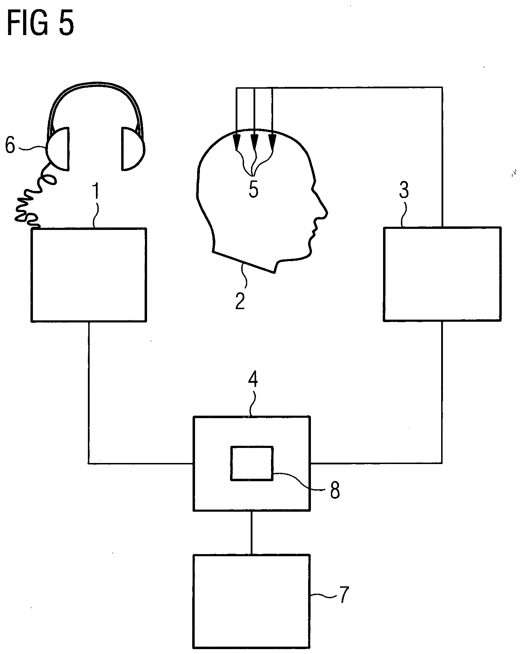

**Fig. 5** zeigt ein Blockschaltbild einer Anordnung zur Analyse von evozierten Potenzial-Signalen und ereigniskorrelierten Potenzial-Signalen einer neuronalen Aktivität. Mit einer Stimuli-Generatoreinheit 1 werden akustische Reize (Stimuli) einem Testsubjekt 2 (Mensch oder Tier) beispielsweise mittels Kopfhörern 6 dargeboten. Mit einer EEG-Einheit, umfassend eine Signalerfassungseinheit 3 mit Sensoren 5, wird die neuronale Aktivität des Gehirns aufgrund des dargebotenen Reizes erfasst (sogenannte Ableitungen der EKPs).

Mit einer eine Klassierungseinheit 8 aufweisenden Rechen- und Steuereinheit 4 wird das oben beschriebene Verfahren zur Analyse mittels Klassieren durchgeführt. Auf einem Bildschirm 7 können die CCR- und MCR-Funktionen sowie die Hörschwelle HS dargestellt werden.

Ein weiterer wichtiger Aspekt von Hörmessungen sind Aussagen über die Hörbahnreifung. Von Neugeborenen ist bekannt, dass die Hörbahnreifung teilweise noch nicht abgeschlossen ist (z.B. bei frühgeborenen Kinder). Diese haben erhöhte Hörschwellen, die sich aber normalisieren. Eine Möglichkeit eine solche Hörbahnreifung oder Plastizität nachzuweisen, liegt in der Erkenntnis, dass auf der rechten Seite ausgelöste Hörereignisse sich am linken Mastoid reproduzieren lassen (Hörbahnkreuzung). Nun könnte auch dieser Algorithmus dazu genutzt werden, zu ermitteln, wie gut sich ipsilateral ausgelöste Potenziale kontralateral reproduzieren lassen. Auch diese Möglichkeit erschließt eine ganz neue Topodiagnostik.

Durch die Erfindung können Auffälligkeiten einfach erkannt werden, wie beispielsweise am Einbruch der CCR- und MCR-Funktionen bei ca. 45 dB/SPL in **Fig. 4** zu sehen ist.

Wenn der Sättigungswert der CCR- und MCR-Funktionen nicht bei 100% liegt, kann dies als Hinweis auf Beeinträchtigung der Hörfähigkeit trotz evtl. normaler Hörschwelle sein, ggf. somit auch ein Hinweis auf eine neuronale Störung der Reizweiterleitung, wie sie z.B. bei einer multiplen Sklerose vorkommt oder sich Hinweise auf eine neuronale Hyperaktivität erweisen, wie sie beim Tinnitussyndrom vorkommen.

Aus der Häufigkeit bei einem bestimmten Lautstärkepegel kann bereits abgeschätzt werden, wie weit man sich von der Hörschwelle HS entfernt befindet (da dort ja gerade der statistische Wert 50% Zuordnung angenommen wird). Dies ermöglicht eine zeitoptimierte Messpunktverteilung bei einer Messung (z.B. verkürzte Narkosedauer, durch weglassen von weiteren Messungen).

Beim Einsatz im Hörscreening kann standardmäßig die Hörschwelle (d.h. der Grad der Schwerhörigkeit) bestimmt werden, anstatt wie bisher nur die Aussage "Patient hört/hört nicht" bei einer einzigen Lautstärke.

Veränderungen können quantifiziert werden, um beispielweise objektiv gutachterlich einen Tinnitus zu erkennen oder zu prüfen, ob der Einsatz von Hörgeräten einen Sinn macht. Auch eine Früherkennung von schädlichen Veränderungen durch zum Beispiel ototoxische Medikamente ist mit einer adaptierten Reizfrequenz möglich.

Das Analyseverfahren bietet auch die Möglichkeit, das Verfahren entweder auf das komplette Potenzial-Signal anzuwenden, oder aber nur bestimmte Teile, wie zum Beispiel die Welle V, die in der Regel zur manuellen Bestimmung der Hörschwelle herangezogen wird, oder nur auf die Welle I, deren Ausprägung mit dem Sprachverstehen korreliert.

Nach bekannter Methodik kann beispielsweise ein Patient als normalhörend eingestuft werden, obwohl er Probleme mit dem Sprachverstehen insbesondere im Störgeräusch hat. D.h. die Hörfähigkeit kann nun einfacher differenziert werden bezüglich der Frage, worauf sie sich genau bezieht, indem z.B. das Verfahren einfach für beide Wellen angewandt wird, was bislang wegen des Auswertungsaufwands kaum praktiziert wird.

Vor der Anwendung des Verfahrens können die Potenzial-Signale durch bekannte Filterungsverfahren verändert werden, um z.B. das Rauschen zu reduzieren (z.B. Binning, etc.).

Alternative Methoden der mathematischen Auswertung / Definition der Hörschwelle aus der Wachstums- / Klassifikationskurve können auch angewandt werden: zum Beispiel bei Abweichung von +- 5% (= Toleranz) von dem statistischen Wert 50% als Kriterium für das Erreichen der Hörschwelle, da bei verrauschten Daten ein Ausreißer oberhalb oder unterhalb der "echten" Hörschwelle gelegentlich eine fehlerhafte Auswertung verursachen könnte.

Eine Alternative kann beispielsweise auch ein polynomialer Fit sein, wobei das Maximum / Minimum der 1. oder 2. Ableitung die Hörschwelle darstellt, oder ein Fit mit einer verschobenen Aufladefunktion oder ein Fit mit einer Sigmoidfunktion.

Die Einteilung der gemessenen Daten (= Potenzial-Signale) in Trainingsdaten (für die Vergleichsfunktion) und Testdaten erfolgt durch hinlänglich bekannte verfahren, wie:

- *K fold:* die Sweeps werden aufgeteilt in k (möglichst gleichgroße) Teilsätze. Jeweils ein Teilsatz wird als Testdatensatz benutzt, die anderen Teilsätze bilden zusammen den Trainingsdatensatz.

- *Hold out:* zufällige Zuordnung von p*100% der Daten in die Testdaten. Typisch p=0.1.

- *Leave 1 out:* jeweils ein Sweep kommt zu den Testdaten, die jeweils anderen kommen zu den Trainingsdaten. Leave 1 Out ist identisch mit k-fold, wenn k = als Anzahl der Beobachtungen gewählt wird.

- *Resubstitution:* alle Sweeps werden sowohl als Trainings- wie auch als Testdaten benutzt. Hierbei besteht jedoch die Gefahr des Overfittings.

Auch folgende bekannte Verfahren können beispielsweise für das Klassieren verwendet werden:

- *SupportVektorMachine:* Nutzung von Vektoren im n-dimensionalen Raum. Dies kann beispielhaftes wie folgt beschrieben werden. Es wird eine Zuordnungsfunktion $f(x, \alpha): x_i \to y_i$ gesucht, die durch den unbekannten Vektor $\alpha$ charakterisiert wird. Der dabei entstehende Zuordnungsfehler des Erwartungswertes soll minimiert sein. Aus dieser Verbundverteilung mit $N$ Beobachtungen werden $L$ Einzelbeobachtungen zufällig bestimmt und als Lerndatensatz genutzt.

$$\{(x_i, y_i) \in Q(x, y)\}\, i = 1 \cdot L\ mit: x_i \in R^N, y_i \in \{+1, -1\} \tag{1}$$

Durch den gewählten Ansatz wird ein Optimum aus dem empirischen Risiko und der Generaliersierungfähigkeit geschaffen. Darüber hinaus werden bei der Trennung optimal angepasste Grenzen berücksichtigt, durch deren Einsatz das komplette Verfahren stabilisiert wird.

- *Decision Tree:* Entscheidungsbäume, deren Binärfragen trainiert werden.

- *(convolutional) Neural Networks:* Neuronale Netze bilden "künstliche Gehirne"

- *Naive bayes estimator:* Berechnung von mehrdimensionalen Wahrscheinlichkeitsdichten

Obwohl die Erfindung im Detail durch die Ausführungsbeispiele näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen.

Bezugszeichenliste

1    Stimuli-Generatoreinheit
2    Testsubjekt
3    Signalerfassungseinheit
4    Rechen- und Steuereinheit
5    Sensor
6    Sensor
7    Bildschirm
8    Klassierungseinheit

A       Signalstärke
$CCR_{A, N}$   Correct Classification Rate Funktion
h       relative Häufigkeit
$MCR_{A, N}$   MisClassification Rate Funktion
HS      Hörschwelle
RP      Schalldruckpegel
S1      ansteigender Bereich
S2      gesättigter Bereich
S3      unterschwelliger Bereich
t       Zeit

I       peripherer Anteil des Nervus chochleans
II      zentraler Anteil des Nervus chochleans
III     Anteil des Nucleus chochleans
IV      Anteil des Nucleus olivaris sauperior
V       Anteil des Colliculi inferiores

**Patentansprüche**

1. Automatisiertes Verfahren zur Verringerung eines Störsignaleinflusses bei der Analyse von evozierten Potenzial-Signalen oder ereigniskorrelierten Potenzial-Signalen einer neuronalen Aktivität, mit den Schritten:

   - Darbieten von physiologischen Reizen einem Testsubjekt (2), wobei der physiologische Reiz durch mindestens einen Parameter (RP) beschreibbar ist,
   - Ermitteln von abgeleiteten zeitabhängigen Potenzial-Signalen,
   - Definieren einer ersten und/oder zweiten Klasse,
   - Zuordnen der Potenzial-Signale oder Teilabschnitten der Potenzial-Signale zu der ersten Klasse oder der zweiten Klasse durch eine Klassierungseinheit,
   - Ermitteln von Häufigkeiten (h) der der ersten Klasse richtig zugeordneten und/oder der zweiten Klasse richtig zugeordneten Potenzial-Signale (CCR) oder zugehörigen Teilabschnitten der Potenzial-Signale und/oder
   - Ermitteln von Häufigkeiten der der ersten Klasse falsch zugeordneten und/oder der zweiten Klasse falsch zugeordneten Potenzial-Signale (MCR) oder zugehörigen Teilabschnitten der Potenzial-Signale.

2. Automatisiertes Verfahren zur Verringerung eines Störsignaleinflusses bei der Analyse von evozierten Potenzial-Signalen oder ereigniskorrelierten Potenzial-Signalen einer neuronalen Aktivität, mit den Schritten:

   - Darbieten von physiologischen Reizen einem Testsubjekt (2), wobei der physiologische Reiz durch mindestens einen Parameter beschreibbar ist,

- Ermitteln von abgeleiteten zeitabhängigen Potenzial-Signalen,
- Definieren einer ersten und/oder zweiten Klasse,
- Zuordnen der Potenzial-Signale oder Teilabschnitten der Potenzial-Signale zu der ersten Klasse oder der zweiten Klasse durch eine Klassierungseinheit und
- Ermitteln von Häufigkeiten der der ersten Klasse zugeordneten und/oder der zweiten Klasse zugeordneten Potenzial-Signale oder zugehörigen Teilabschnitten der Potenzial-Signale.

3. Verfahren nach Anspruch 1 oder 2,
   **gekennzeichnet durch:**

   - Bereitstellen einer ersten Vergleichsfunktion,
   - wobei die erste Klasse als zur Klasse der ersten Vergleichsfunktion gehörig und die zweite Klasse als nicht zur Klasse der ersten Vergleichsfunktion oder zur Klasse einer zweiten Vergleichsfunktion gehörig definiert ist.

4. Verfahren nach einem der Ansprüche 1 bis 3,
   **dadurch gekennzeichnet,**
   **dass** die zweite Vergleichsfunktion eine stochastischen Zufallsverteilungsfunktion oder eine konstante Funktion gleichen Mittelwerts ist.

5. Verfahren nach einem der vorhergehenden Ansprüche,
   **gekennzeichnet durch:**

   - wiederholtes Durchführen des Verfahrens nach einem der Ansprüche 1 bis 4, wobei der Wert des Parameters (RP) vor jedem wiederholten Durchführen verändert wird.

6. Verfahren nach Anspruch 5,
   **gekennzeichnet durch:**

   - Darstellen der ermittelten Häufigkeiten (h) in Abhängigkeit des Wertes des Parameters (RP).

7. Verfahren nach Anspruch 5,
   **gekennzeichnet durch:**

   - Ermitteln eines Schwellwerts (HS) des Parameters (RP), ab dem die Häufigkeiten (h) von einem vorgebbaren Häufigkeitsschwellwert oder um eine vorgebbare Toleranz um den Häufigkeitsschwellwert abweichen.

8. Verfahren nach Anspruch 7,
   **dadurch gekennzeichnet,**
   **dass** der Häufigkeitsschwellwert 50 % ist.

9. Verfahren nach einem der Ansprüche 3 bis 8,
   **dadurch gekennzeichnet,**
   **dass** die erste Vergleichsfunktion aus mindestens einem Potenzial-Signal des Testsubjekts (2) ermittelt wird.

10. Verfahren nach einem der Ansprüche 3 bis 9,
    **dadurch gekennzeichnet,**
    **dass** die erste und/oder zweite aus Vergleichsfunktion mindestens einem Potenzial-Signal des Testsubjekts (2) ohne Darbietung eines physiologischen Reizes ermittelt wird.

11. Verfahren nach einem der Ansprüche 3 bis 9,
    **dadurch gekennzeichnet,**
    **dass** die erste und/oder zweite Vergleichsfunktion aus Potenzial-Signalen mindestens eines weiteren Testsubjekts (2) ermittelt wird.

12. Verfahren nach einem der Ansprüche 3 bis 11:

    **dadurch gekennzeichnet,**
    **dass** die erste und/oder zweite Vergleichsfunktion durch Mittelwertbildung von Potenzial-Signalen oder durch Verfahren der künstlichen Intelligenz ermittelt wird.

**13.** Verfahren nach einem der Ansprüche 3 bis 12,
**dadurch gekennzeichnet,**
**dass** das Klassieren entsprechend der kleinsten Summe der Fehlerquadrate bezogen auf die erste Vergleichsfunktion und die zweite Vergleichsfunktion erfolgt.

**14.** Verfahren nach einem der vorhergehenden Ansprüche,
**dadurch gekennzeichnet,**
**dass** der physiologische Reiz ein akustischer Reiz ist.

**15.** Verfahren nach Anspruch 14,
**dadurch gekennzeichnet,**
**dass** der Parameter ein Schalldruckpegel (RP) und der Schwellwert eine Hörschwelle (HS) ist.

**16.** Anordnung zur Verringerung eines Störsignaleinflusses bei der Analyse von evozierten Potenzial-Signalen oder ereigniskorrelierten Potenzial-Signalen einer neuronalen Aktivität, aufweisend:

- eine Stimuli-Generatoreinheit (1), die ausgebildet ist, mindestens einen physiologischen Reiz darzubieten,
- eine Signalerfassungseinheit (3) mit mindestens einem Sensor (5), die ausgebildet ist, eine neuronale Aktivität aufgrund des physiologischen Reizes zu erfassen, und
- eine eine Klassierungseinheit (8) aufweisende Rechen- und Steuereinheit (4), die ausgebildet und programmiert ist, das Verfahren nach einem der Ansprüche 1 bis 15 auszuführen.

**17.** Computerlesbares Medium, auf welchem ein Computerprogramm gespeichert ist, wobei das Computerprogramm in eine Speichereinrichtung einer Einrichtung ladbar ist, wobei mit dem Computerprogramm die Schritte eines Verfahrens nach einem der Ansprüche 1 bis 15 ausgeführt werden, wenn das Computerprogramm auf der Einrichtung ausgeführt wird.

FIG 1

FIG 2

FIG 3

FIG 4

# FIG 5

Europäisches
Patentamt

European
Patent Office

Office européen
des brevets

**EUROPÄISCHER RECHERCHENBERICHT**

Nummer der Anmeldung

EP 18 00 0549

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2014/052938 A1 (UNIV CALIFORNIA [US]; SALK INST FOR BIOLOGICAL STUDI [US]) 3. April 2014 (2014-04-03) * Seite 2, Zeilen 4, 16 * * Seite 3, Zeile 34 * * Seite 4, Zeilen 6, 7 * * Seite 4, Zeile 15 - Seite 5, Zeile 28 * * Seite 10, Zeile 15 * * Seite 14, Zeilen 4, 6, 8-10 * * Seite 20, Zeilen 24-26 * * Seite 21, Zeile 1 * * Seite 30, Zeilen 2, 4, 6, 7, 9, 10, 18, 19 * * Seite 31, Zeilen 5, 6, 17 * * Seite 32, Zeilen 4, 5, 21 * * Abbildungen 1, 1A, 5A, 5B, 6, 11-13 * ----- | 1-17 | INV. A61B5/12 A61B5/0484 A61B5/00 ADD. A61B5/16 |
| A | WO 2014/138925 A1 (INTERAXON INC [CA]) 18. September 2014 (2014-09-18) * Seite 64, Zeile 24 - Seite 65, Zeile 25 * ----- | 6-8 | |
| A | WO 2016/046830 A2 (ELMINDA LTD [IL]) 31. März 2016 (2016-03-31) * Seite 93, Zeile 3 - Seite 94, Zeile 13 * ----- | 13 | RECHERCHIERTE SACHGEBIETE (IPC) A61B |
| A | PAULRAJ M P ET AL: "A machine learning approach for distinguishing hearing perception level using auditory evoked potentials", 2014 IEEE CONFERENCE ON BIOMEDICAL ENGINEERING AND SCIENCES (IECBES), IEEE, 8. Dezember 2014 (2014-12-08), Seiten 991-996, XP032738973, DOI: 10.1109/IECBES.2014.7047661 [gefunden am 2015-02-23] * Seite 993, linke Spalte, Absatz 1 * ----- -/-- | 15 | |

Der vorliegende Recherchenbericht wurde für alle Patentansprüche erstellt

| Recherchenort | Abschlußdatum der Recherche | Prüfer |
|---|---|---|
| Den Haag | 15. Oktober 2018 | Meyer, Wolfgang |

Seite 1 von 2

Europäisches
Patentamt

European
Patent Office

Office européen
des brevets

# EUROPÄISCHER RECHERCHENBERICHT

Nummer der Anmeldung

EP 18 00 0549

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2006/009771 A1 (NEURONETRIX INC [US]; FADEM KALFORD C [US]) 26. Januar 2006 (2006-01-26) * das ganze Dokument * ----- | 1-17 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 15. Oktober 2018 | Meyer, Wolfgang |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

..............................................................................
& : Mitglied der gleichen Patentfamilie, übereinstimmendes
Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 18 00 0549

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-10-2018

| Im Recherchenbericht angeführtes Patentdokument | Datum der Veröffentlichung | Mitglied(er) der Patentfamilie | Datum der Veröffentlichung |
|---|---|---|---|
| WO 2014052938 A1 | 03-04-2014 | BR 112015007002 A2 | 04-07-2017 |
| | | CA 2886095 A1 | 03-04-2014 |
| | | CN 104871160 A | 26-08-2015 |
| | | EP 2901342 A1 | 05-08-2015 |
| | | JP 2015533559 A | 26-11-2015 |
| | | KR 20150076167 A | 06-07-2015 |
| | | US 2015248470 A1 | 03-09-2015 |
| | | US 2018285442 A1 | 04-10-2018 |
| | | WO 2014052938 A1 | 03-04-2014 |
| WO 2014138925 A1 | 18-09-2014 | CA 2942852 A1 | 18-09-2014 |
| | | EP 2972678 A1 | 20-01-2016 |
| | | US 2014347265 A1 | 27-11-2014 |
| | | WO 2014138925 A1 | 18-09-2014 |
| WO 2016046830 A2 | 31-03-2016 | CA 2961451 A1 | 31-03-2016 |
| | | EP 3197350 A2 | 02-08-2017 |
| | | US 2017216595 A1 | 03-08-2017 |
| | | WO 2016046830 A2 | 31-03-2016 |
| WO 2006009771 A1 | 26-01-2006 | AU 2005265033 A1 | 26-01-2006 |
| | | EP 1781165 A1 | 09-05-2007 |
| | | EP 2260760 A1 | 15-12-2010 |
| | | JP 4833202 B2 | 07-12-2011 |
| | | JP 2008503261 A | 07-02-2008 |
| | | US 2007191727 A1 | 16-08-2007 |
| | | US 2014128763 A1 | 08-05-2014 |
| | | WO 2006009771 A1 | 26-01-2006 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102014007647 A1
- WO 2015161343 A1
- WO 2008065239 A1